# EUROPÄISCHE PATENTSCHRIFT

(11) **EP 0 641 764 B1**
(45) Veröffentlichungstag und Bekanntmachung des Hinweises auf die Patenterteilung: **20.05.1998**
(21) Anmeldenummer: 94112791.2
(22) Anmeldetag: 17.08.1994
(51) Int. Cl.: C07C 213/00, C07C 215/16

(54) **Verfahren zur Herstellung von Hydroxyalkylaminonitrobenzol-Derivaten**
Process for the preparation of hydroxyalkylaminonitrobenzene derivatives
Procédé pour la préparation de dérivés d'hydroxyalkylaminonitrobenzène

(30) Priorität: 03.09.1993 DE 4329727
(43) Veröffentlichungstag der Anmeldung: 08.03.1995
(73) Patentinhaber: Clariant GmbH, 65929 Frankfurt am Main (DE)
(72) Erfinder: Bauer, Wolfgang, Dr., D-63477 Maintal (DE); Delpy, Klaus, Dr., D-61328 Dietzenbach (DE); Bittner, Andreas, Dr., D-63071 Offenbach/Main (DE)

(56) Entgegenhaltungen:
- DE-A- 2 717 766
- DE-A- 3 943 545
- GB-A- 2 165 258
- GB-A- 2 216 124
- GB-A- 2 232 419

## Beschreibung

Die vorliegende Erfindung betrifft die Herstellung von Hydroxyalkylaminonitrobenzolderivaten durch Umsetzung von Nitroanilinderivaten mit Chloralkylchlorformiaten und Umlagerung der erhaltenen Carbamate zu den Zielverbindungen.

Hydroxyalkylaminonitrobenzolderivate sind wichtige Zwischenprodukte zur Herstellung bedeutender Farbstoffe zur Färbung von Haaren und Pelzen.

Zu ihrer Herstellung sind bereits eine Reihe von Verfahren publiziert worden, die aber allesamt sowohl in ökonomischer als auch in ökologischer Hinsicht nicht zufriedenstellen. Beispielsweise ist es bereits bekannt, Nitroanilinderivate mit Chloralkylchlorformiaten zu den entsprechenden Carbamaten umzusetzen, diese zu isolieren und gegebenenfalls zu reinigen und dann durch Basenbehandlung über die entsprechenden Oxazolidinone in die Zielverbindungen umzulagern (J. Am. Chem. Soc. 45, 785 (1923), Farmaco Ed. Sci. 24, 179 (1969), DE-A-38 06 237, DE-A-38 44 517, DE-A 39 17 113, DE-A-33 48 135 und DE-A-27 17 766).

Die Umsetzung zum Carbamat wird dabei normalerweise in inerten, aprotischen Lösungsmitteln (beispielsweise Benzol, Aceton, Dioxan, Pyridin, Monoethylenglykoldimethylether) oder auch ohne Lösungsmittel in Gegenwart eines Überschusses an aromatischem Amin oder einer Base, wie Calciumcarbonat oder Triethylamin, durchgeführt. Isolierung und gegebenenfalls Reinigung durch Umkristallisation schließen sich an.

Bei den ohne Lösungsmittel arbeitenden Verfahren werden die Edukte auf Temperaturen bis 130°C erhitzt, was aus sicherheitstechnischen Gründen Probleme aufwirft. Bei den mit Lösungsmitteln arbeitenden Verfahren fallen nach der Produktisolierung Filtrate und Abwässer an, aus denen die eingesetzten Lösungsmittel in aufwendiger Weise regeneriert werden müssen, um eine Belastung der Umwelt zu vermeiden.

Gemäß Stand der Technik werden die derart hergestellten Carbamate unter Einsatz eines weiteren Lösungsmittels, beispielsweise Methanol oder Ethanol, mit starken Basen, wie Natrium- oder Kaliumhydroxid oder Natriummethylat, umgelagert, wobei gemäß US-A-4 910 341 sogar noch die Oxazolidinon-Zwischenstufe isoliert wird.

Üblicherweise enthalten die derart unter Verwendung von Alkoholen hergestellten Produkte Verunreinigungen in Form von Aminonitrobenzolderivaten, die durch unerwünschte hydrolytische Spaltung der Carbamate entstehen. Somit sind zur Herstellung von Produkten hoher Reinheit aufwendige und kostenintensive Reinigungsoperationen, beispielsweise mehrfache Umkristallisation, notwendig. Damit verbunden sind Ausbeuteverluste, hohe Umweltbelastung und somit eine deutliche Verteuerung des Verfahrens.

Aufgabe der vorliegenden Erfindung ist es, ein in ökonomischer und ökologischer Hinsicht vorteilhafters Verfahren zur Herstellung von Hydroxyalkylaminonitrobenzol-Derivaten bereitzustellen.

Überraschenderweise wurde nun gefunden, daß die Carbamatzwischenstufe ohne Isolierung, d.h. auch ohne Einsatz eines zweiten Lösungsmittels weiter umgesetzt werden kann, wobei ein hochreines Produkt erhalten wird.

Die vorliegende Erfindung betrifft somit ein Verfahren zur Herstellung von Hydroxyalkylaminonitrobenzol-Derivaten der allgemeinen Formel I 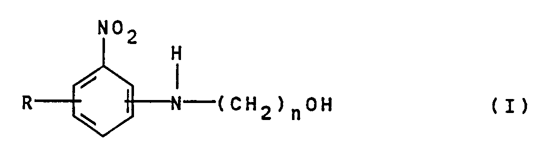 worin
n 2 oder 3 und
R Wasserstoff, Hydroxy, Halogen, (C₁-C₄)-Alkyl, (C₁-C₄)-Alkoxy, Amino, Mono-(C₁-C₄)-alkylamino, Di-(C₁-C₄)-alkylamino oder (C₁-C₄)-alkylcarbonylamino
bedeuten,
durch Umsetzung von Verbindungen der allgemeinen Formel II 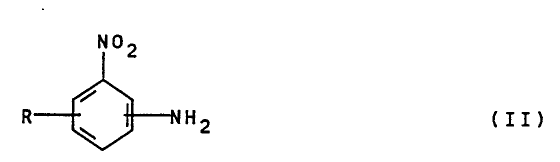 worin R wie oben angegeben definiert ist mit Verbindungen der allgemeinen Formel III 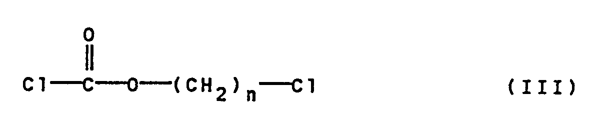 worin n wie oben angegeben definiert ist, in inerten aprotischen Lösungsmitteln in Gegenwart einer Base zu Verbindungen der allgemeinen Formel IV 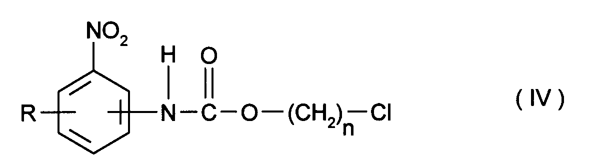 worin R und n wie oben angegeben definiert sind und Umlagerung der Verbindung der allgemeinen Formel IV mit einem Alkalihydroxid, dadurch gekennzeichnet, daß die Verbindung der allgemeinen Formel IV nicht zwischenisoliert wird und daß nach erfolgter Umsetzung der Verbindung der allgemeinen Formel II mit der Verbindung der allgemeinen Formel III Wasser zugegeben, die salzhaltige Wasserphase bei pH-Werten von 0 bis 7 abgetrennt und die organische Phase weiter zur Verbindung der allgemeinen Formel I umgesetzt wird.

In der allgemeinen Formel I steht die Hydroxyalkylaminogruppe bevorzugt in ortho- oder meta-Stellung zur Nitrogruppe.

Der Substituent R kann jede der vier freien Positionen am Benzolkern besetzen.

Besonders bevorzugt sind Verbindungen der allgemeinen Formel Ia 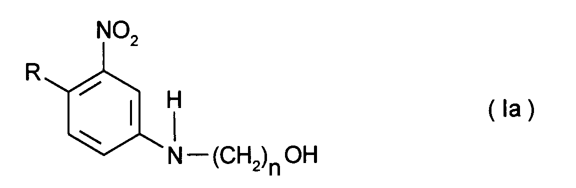 worin n und R wie oben angegeben definiert sind.

Analoges gilt jeweils für die Verbindungen der allgemeinen Formeln II und IV.

Für R stehendes (C₁-C₄)-Alkyl kann geradkettig oder verzweigt sein und bedeutet Methyl, Ethyl, n-Propyl, i-Propyl, n-Butyl, i-Butyl, sek.-Butyl oder tert.-Butyl. Analoges gilt für die für R stehenden Alkoxy-, Monoalkylamin-, Dialkylamin- und Alkylcarbonylaminogruppen. Halogen bedeutet insbesondere Fluor, Chlor, Brom oder Iod. Bevorzugte Reste R sind Wasserstoff, Methyl, Methoxy, Ethoxy, Hydroxy und Chlor.

Erfindungsgemäß einzusetzende inerte aprotische Lösungsmittel sind bevorzugt Diethoxymethan, Diethylenglykoldimethylether, Triethylenglykoldimethylether, Dioxan und Tetrahydrofuran. Besonders bevorzugt ist Monoethylenglykoldiethylether und ganz besonders bevorzugt sind Monoethylenglykoldimethylether und Methyl-tert.-butylether.

Die inerten aprotischen Lösungsmittel können auch Wasser in Mengen von 0 bis 30 Gew.% enthalten, wobei 1 bis 10 Gew.% besonders bevorzugt sind.

Als zur Umsetzung der Verbindungen der allgemeinen Formeln II und III notwendige Base können Alkalicarbonate, Alkalibicarbonate, Erdalkalicarbonate, Erdalkalibicarbonate, Alkalihydroxide, Erdalkalihydroxide, Erdalkalioxide oder tertiäre Amine eingesetzt werden. Bevorzugt sind Erdalkalicarbonate, wie Calcium- und Magnesiumcarbonat. Besonders bevorzugt sind Erdalkalihydroxide, wie Calcium- und Magnesiumhydroxid, sowie Erdalkalioxide, wie Calcium- und Magnesiumoxid.

Die genannten Basen werden bevorzugt in Mengen von 0,5 bis 0,7 Mol, besonders bevorzugt in Mengen von 0,5 bis 0,6 Mol, pro Mol Verbindung der allgemeinen Formel II eingesetzt.

Die Verbindungen der allgemeinen Formel III werden bevorzugt in Mengen von 1 bis 1,4 Mol, besonders bevorzugt in Mengen von 1 bis 1,2 Mol, pro Mol Verbindung der allgemeinen Formel II eingesetzt.

Bei der Umsetzung der Verbindungen der allgemeinen Formeln II und III wird üblicherweise ein Temperaturbereich von 20 bis 120°C eingehalten, wobei 50°C bis 90°C bevorzugt sind. Der pH-Wert liegt bei dieser Umsetzung bevorzugt zwischen 3 und 10, besonders bevorzugt zwischen 4 und 8.

Die zur Umlagerung der Verbindungen der allgemeinen Formel IV notwendigen Alkalihydroxide sind bevorzugt Lithium-, Natrium- und Kaliumhydroxid, die auch in Mischungen untereinander eingesetzt werden können.

Die genannten Alkalihydroxide werden bevorzugt in Mengen von 2,5 bis 4,5 Mol, besonders bevorzugt in Mengen von 3,0 bis 4,0 Mol und ganz besonders bevorzugt in Mengen von 3,3 bis 3,7 Mol, jeweils pro Mol Verbindung der allgemeinen Formel IV, eingesetzt.

Die genannten Alkalihydroxide können in fester Form, aber bevorzugt in Form wäßriger Lösungen, beispielsweise in Form 50 %iger wäßriger Lösungen, eingesetzt werden.

Die Umlagerungsreaktion wird bevorzugt in einem Temperaturbereich von 10 bis 120°C, bevorzugt in einem Temperaturbereich von 40 bis 90°C durchgeführt.

Das erfindungsgemäße Verfahren kann auch in Gegenwart von anionischen, kationischen oder nichtionogenen Tensiden oder von Phasentransferkatalysatoren ausgeführt werden.

Beispiele für solche Verbindungen sind quartäre Ammoniumverbindungen wie Octadecyltrimethylammoniumchlorid, Didecyldimethylammoniumchlorid oder Trioctylmethylammoniumchlorid, quartäre Phosphoniumverbindungen wie Tributylhexadecylphosphoniumbromid, Polyethylenglykolether wie Pentaethylenglykoldimethylether oder Polyethylenglykoldimethylether vom Molgewicht 500, 1000 oder 2000, Kronenether wie 18-Krone-6 oder Tris-(3,6-dioxaheptyl)-amin.

Der Verbindungen der genannten Art werden in Mengen von 0,01 bis 5, bevorzugt 0,1 bis 2 Gew.-%, bezogen auf eingesetztes Amin der Formel II, zugesetzt.

Das erfindungsgemäße Verfahren wird beispielsweise so ausgeführt, daß man eine Verbindung der allgemeinen Formel II in einem inerten aprotischen Lösungsmittel löst und wie oben beschrieben mit einer Verbindung der allgemeinen Formel III umsetzt. Nach erfolgter Umsetzung wird Wasser zugegeben und aus dem erhaltenen Zweiphasengemisch die salzhaltige Wasserphase bevorzugt bei pH-Werten von 1 bis 3, abgetrennt. Die organische Phase wird dann direkt weiter zum Endprodukt umgesetzt. Üblicherweist weist diese organische Phase einen Wassergehalt von 0 bis 15 Gew.-%, bevorzugt von 1 bis 10 Gew.-%, auf.

Nach erfolgter Umlagerung zu den Verbindungen der allgemeinen Formel I werden die erhaltenen Lösungen üblicherweise, gegebenenfalls nach Zusatz von Wasser von den alkalischen salzhaltigen Wasserphasen getrennt und durch übliche Aufarbeitungsmethoden, beispielsweise durch Redestillation der eingesetzten inerten Lösungsmittel und Zusatz von Wasser, isoliert. Alternativ kann die Produktisolierung auch aus dem zunächst erhaltenen Zweiphasengemisch ohne Abtrennung der wäßrigen Phase erfolgen. Dabei wird die Redestillation des eingesetzten Lösungsmittels bevorzugt in einem pH-Bereich von 4 bis 10, besonders bevorzugt von 6 bis 9, vorgenommen.

Das erfindungsgemäße Verfahren liefert die Verbindungen der allgemeinen Formel I in sehr guter Ausbeute und in sehr hoher Reinheit, und dies obwohl weder die Zwischenverbindungen der allgemeinen Formel IV noch die Endprodukte beispielsweise durch Umkristallisationsmethoden gereinigt werden. Weitere Vorteile des erfindungsgemäßen Verfahrens im Vergleich zu den bekannten Verfahren ergeben sich auch daraus, daß nur ein, anstatt zwei, Lösungsmittel eingesetzt wird. So sind die Produkte der allgemeinen Formel I mit besserer Raum/Zeit-Ausbeute bei deutlich verringerter Abwasserfracht zugänglich.

In den nachstehenden Beispielen bedeuten Prozentangaben Gewichtsprozent.

### Beispiel 1:

### Herstellung von 2-Hydroxyethylamino-nitrobenzol

Eine Lösung von 138,1 g 2-Nitroanilin in 360 g Monoethylenglykoldimethylether und 60 g Wasser wird mit 60,1 g Calciumcarbonat versetzt. Anschließend dosiert man bei 80°C 150,0 g 2-Chlorethylchlorformiat zu und vervollständigt die Reaktion durch Nachrühren bei 80°C. Dann gibt man 100 g Wasser und 8,7 g Salzsäure 35 %ig bis zu einem pH-Wert von 1 zu und trennt die untere Wasserphase ab. Die obere organische Phase wird mit 90 g Monoethylenglykoldimethylether verdünnt und bei 15°C mit 370,6 g einer 50 %igen Kaliumhydroxidlösung versetzt. Anschließend heizt man das Reaktionsgemisch auf 85°C und rührt 4 Stunden bei 85°C nach. Darauf werden 250 g Wasser zugegeben und die untere salzhaltige Wasserphase abgetrennt. Die gelbrote organische Phase wird zunächst mit 500 g Wasser und dann mit 9 g Schwefelsäure 10 %ig bis zu einem pH-Wert von 8,5 versetzt. Nach Abdestillieren von Monoethylenglykoldimethylether und Abkühlen auf 10°C erhält man das Produkt in Form gelbroter Kristalle.
Ausbeute: 173,4 g
Reingehalt: 99,8 %
Ausbeute in % d. Theorie, bezogen auf 2-Nitroanilin: 95 %
Gehalt an 2-Nitroanilin: 0,2 %
Schmelzpunkt: 70 - 72°C

### Vergleich: Verfahren gemäß DE-A 38 06 237:

### Stufe 1: Herstellung von 2-Nitrophenyl-2-chlorethylcarbamat

Eine Lösung von 345,3 g 2-Nitroanilin in einem Gemisch von 1020 g Monoethylenglykoldimethylether und 150 g Wasser wird mit 135 g Calciumcarbonat versetzt und auf 78°C geheizt. Anschließend werden in 3 Stunden 375 g 2-Chlorethylchlorformiat zugegeben. Man rührt 1,5 Stunden bei Rückflußtemperatur bis zur vollständigen Umsetzung nach, kühlt auf 35°C und fällt das Produkt mit Wasser und Eis aus. Das Produkt wird abfiltriert, mit Wasser gewaschen und getrocknet.
Ausbeute: 556,5 g schwachgelbe Kristalle
Ausbeute in % d. Theorie, bezogen auf 2-Nitroanilin: 91 %
Schmelzpunkt: 64 - 67°C

### Stufe 2: Herstellung von 2-Hydroxyethylamino-nitrobenzol

244,6 g des nach Stufe 1 erhaltenen Carbamats werden in 900 g Wasser und 236 g Ethanol eingetragen, auf 60°C erhitzt und in 1,5 Stunden mit 340,4 g 50 %iger Kaliumhydroxidlösung versetzt. Man rührt 2,5 Stunden bei 60°C nach, stellt das Reaktionsgemisch mit Eisessig auf pH 8,0 und läßt abkühlen. Das ausgefallene Produkt wird abgesaugt, mit Wasser gewaschen und bei 50°C im Vakuum getrocknet.
Ausbeute: 168,8 g gelbrotes Granulat
Reingehalt: 95 %
Ausbeute in % d. Theorie, bezogen auf Carbamat: 88 %
Ausbeute in % d. Theorie, bezogen auf 2-Nitroanilin: 80,1 %
Gehalt an 2-Nitroanilin: 1,3 %
Schmelzpunkt: 68 - 70°C

### Beispiel 2

### 4-(2-Hydroxyethylamino)-2-nitroanisol

Eine Mischung von 168,2 g 4-Amino-2-nitroanisol, 42,8 g Calciumhydroxid und 5 g Tetraethylenglykoldimethylether in 1500 g wäßrigem Monoethylenglykoldimethylether wird nach den Angaben des Beispiels 1 mit 150 g 2-Chlorethylchlorformiat und nach der Phasentrennung mit 370,6 g Kaliumhydroxid 50 %ig umgesetzt. Nach Aufarbeitung analog Beispiel 1 erhält man gelbrote Kristalle.
Ausbeute: 183,3 g
Reingehalt: 99,9 %
Ausbeute in % d. Theorie: 86,3 %
Gehalt an 4-Amino-2-nitroanisol: 480 ppm
Schmelzpunkt: 82 - 84°C

### Vergleich: Verfahren gemäß DE-A 38 06 237:

### Stufe 1: 2-Chlorethyl-(3-nitro-4-methoxyphenyl)-carbamat

420,5 g 4-Amino-2-nitroanisol werden in 1275 ml Diethylenglykoldimethylether und 150 ml Wasser gelöst, mit 135 g Calciumcarbonat versetzt und auf 78°C geheizt. Zu dieser Mischung läßt man in 3 Stunden 375 g 2-Chlorethylchlorformiat so zulaufen, daß die Reaktionstemperatur bei 78 - 80°C bleibt. Man rührt 1,5 Stunden zur Vervollständigung der Reaktion nach, kühlt auf 35°C und gibt Wasser und Eis zu. Das ausgefällte Produkt wird abfiltriert, mit Wasser gewaschen und getrocknet.
Ausbeute: 663 g
Ausbeute in % d. Theorie, bezogen auf 4-Amino-2-nitroanisol: 96,5 %
Schmelzpunkt: 82 - 84°C

### Stufe 2: 4-Hydroxyethylamino-2-nitroanisol

618 g des nach Stufe 1 erhaltenen Carbamats werden in 2025 ml Wasser und 675 ml Ethanol vorgelegt, auf 60°C erhitzt und in 1,5 Stunden mit 766 g einer 50 %igen Kaliumhydroxidlösung versetzt. Man rührt 2,5 Stunden bei 60°C nach, stellt mit Eisessig auf pH 8 und kühlt ab. Das ausgefallene Produkt wird abfiltriert, mit Wasser gewaschen und bei 50°C im Vakuum getrocknet.
Ausbeute: 423,9 g braune Granulate
Reinheitsgehalt: 93,2 %
Ausbeute in % d. Theorie, bezogen auf 4-Amino-2-nitroanilin: 82,7 %
Gehalt an 4-Amino-2-nitroanisol: 2500 ppm
Schmelzpunkt: 79°C

Um das Produkt in einer Reinheit von > 99 % und einem Gehalt von < 600 ppm 4-Amino-2-nitroanisol zu erhalten, muß das Produkt mehrfach aus wäßrigem Methanol umkristallisiert werden:

### 1. Umkristallisation

188,4 g des nach Vergleichsbeispiel 3 erhaltenen Produkts werden in einem Gemisch aus 270 g Methanol und 590 g Wasser bei 65°C gelöst und mit 4,3 g Aktivkohle versetzt. Anschließend wird filtriert und der Rückstand mit einer Mischung aus 5,5 g Methanol und 12 g Wasser gewaschen. Die filtrierte Lösung wird zur Kristallisation auf 10°C abgekühlt. Nach Filtration und Trocknung erhält man gelbrote Kristalle.
Ausbeute: 162,1 g
Reingehalt: 97,5 %
Ausbeute in % d. Theorien, bezogen auf eingesetztes 4-Amino-2-nitroanisol: 97,5 %
Gehalt an 4-Amino-2-nitroanisol: 1100 ppm
Schmelzpunkt: 80 - 81°C

### 2. Umkristallisation

162,1 g des nach der 1. Umkristallisation erhaltenen Produkts werden aus einer Mischung von 531 g Wasser und 243 g Methanol unter Zusatz von 3,9 g Aktivkohle umkristallisiert. Nach Filtration und Trocknung erhält man gelbrote Kristalle.
Ausbeute: 142,9 g
Reingehalt: 99,5 %
Ausbeute in % d. Theorie, bezogen auf eingesetztes 4-Amino-2-nitroanilin: 67 %
Gehalt an 4-Amino-2-nitroanilin: 550 ppm
Schmelzpunkt: 81 - 83°C

Die folgende Tabelle zeigt weitere erfindungsgemäße Beispiele:

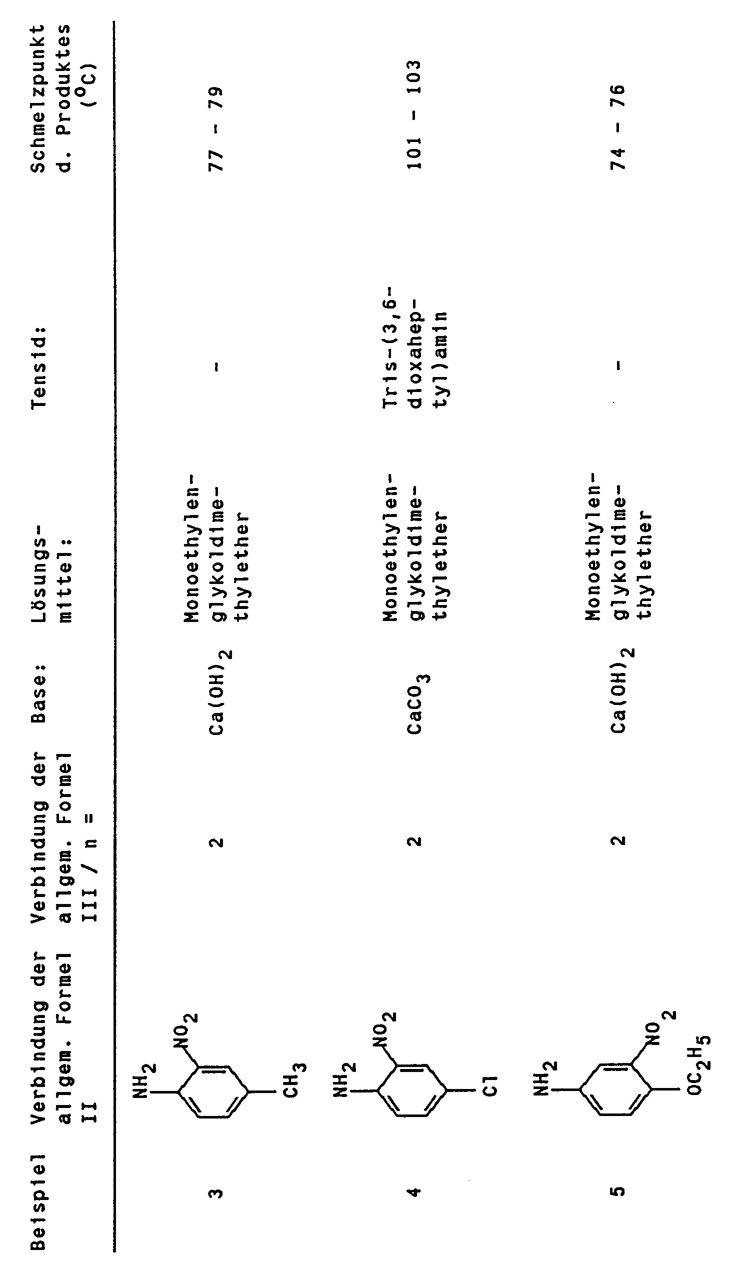

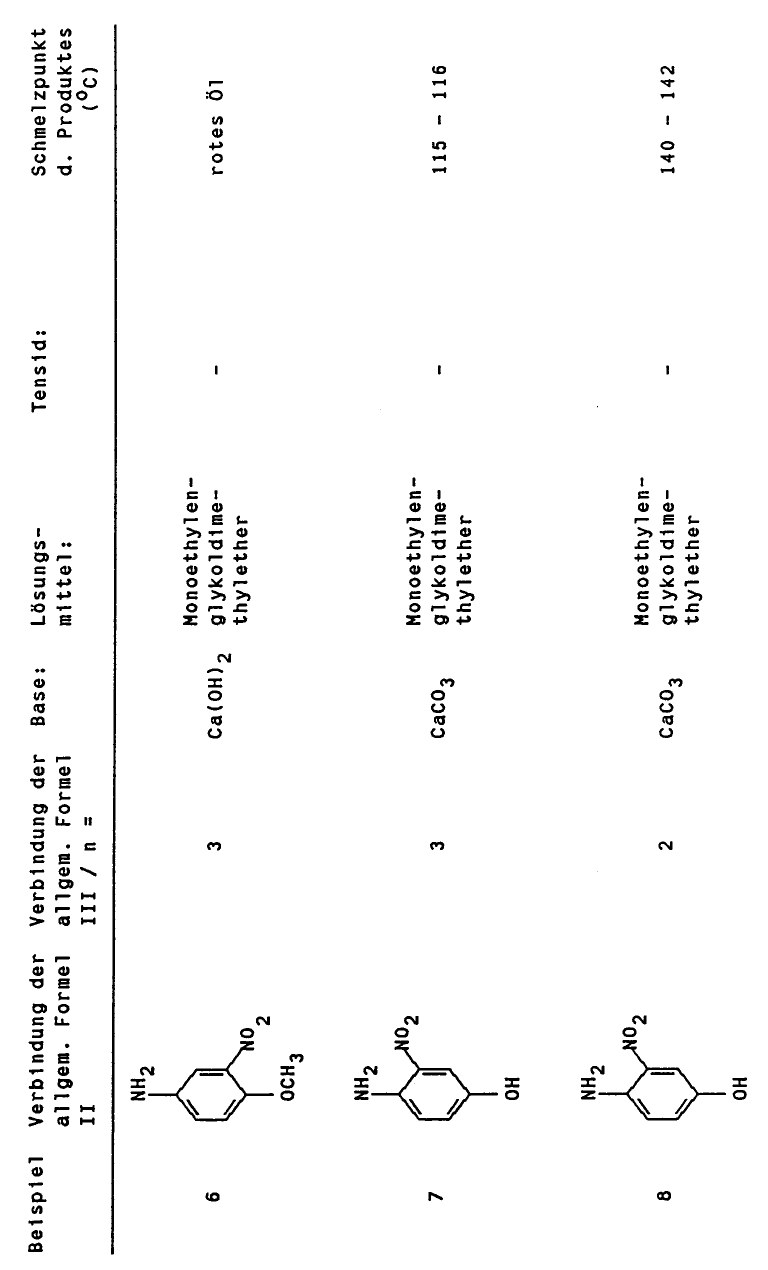

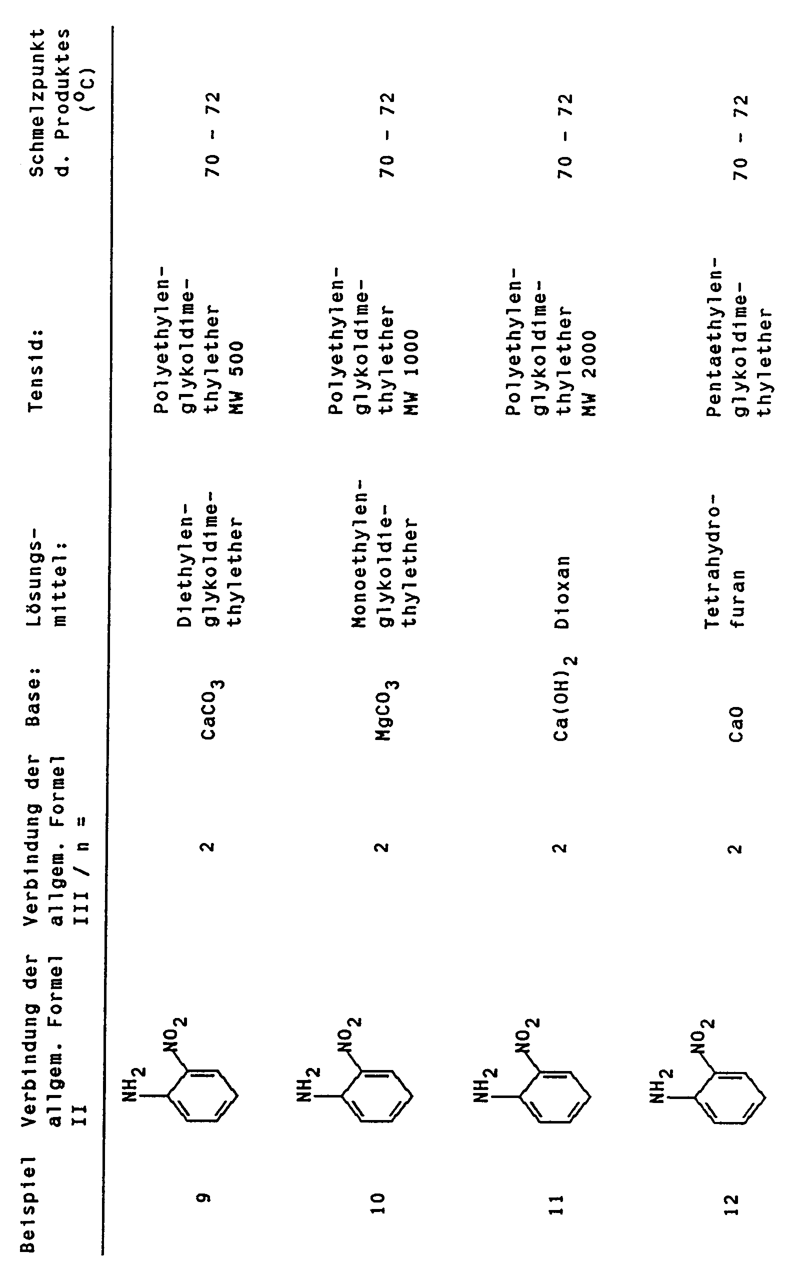

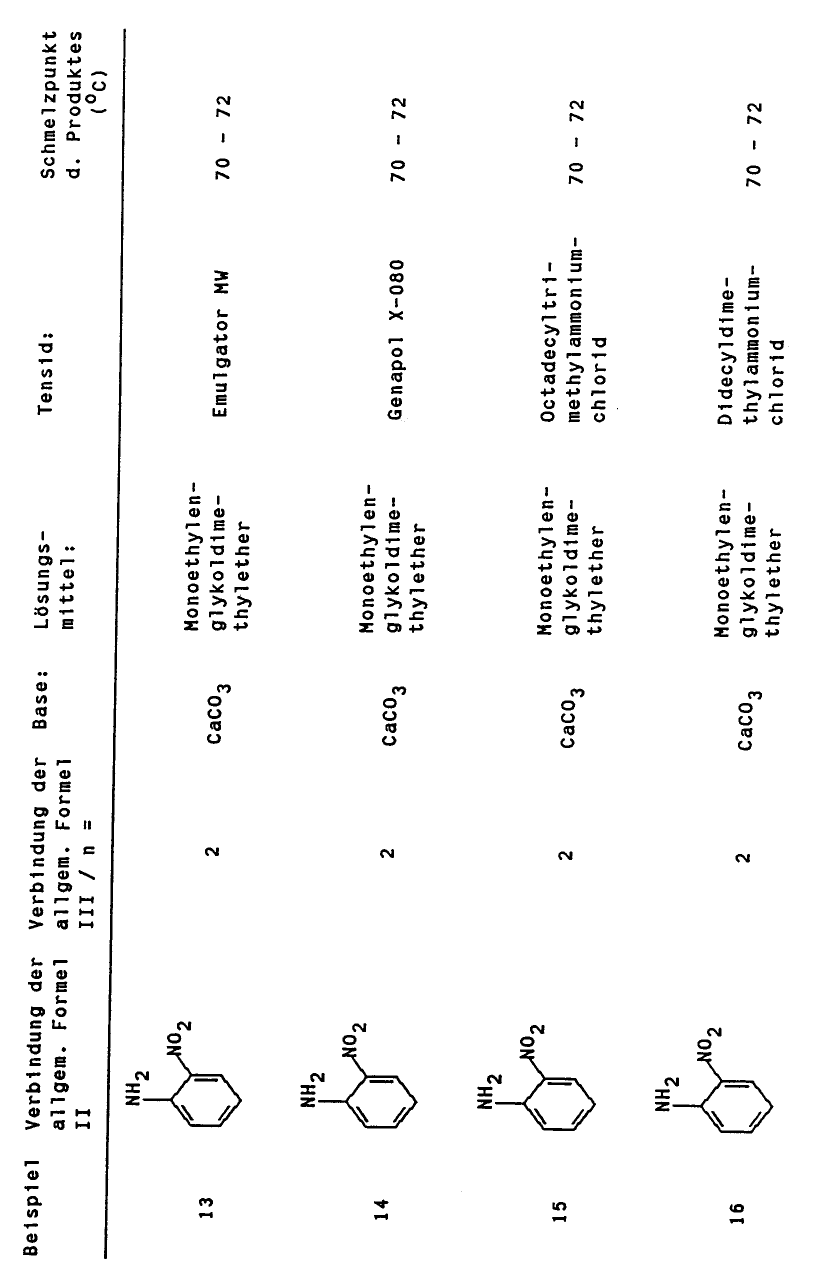

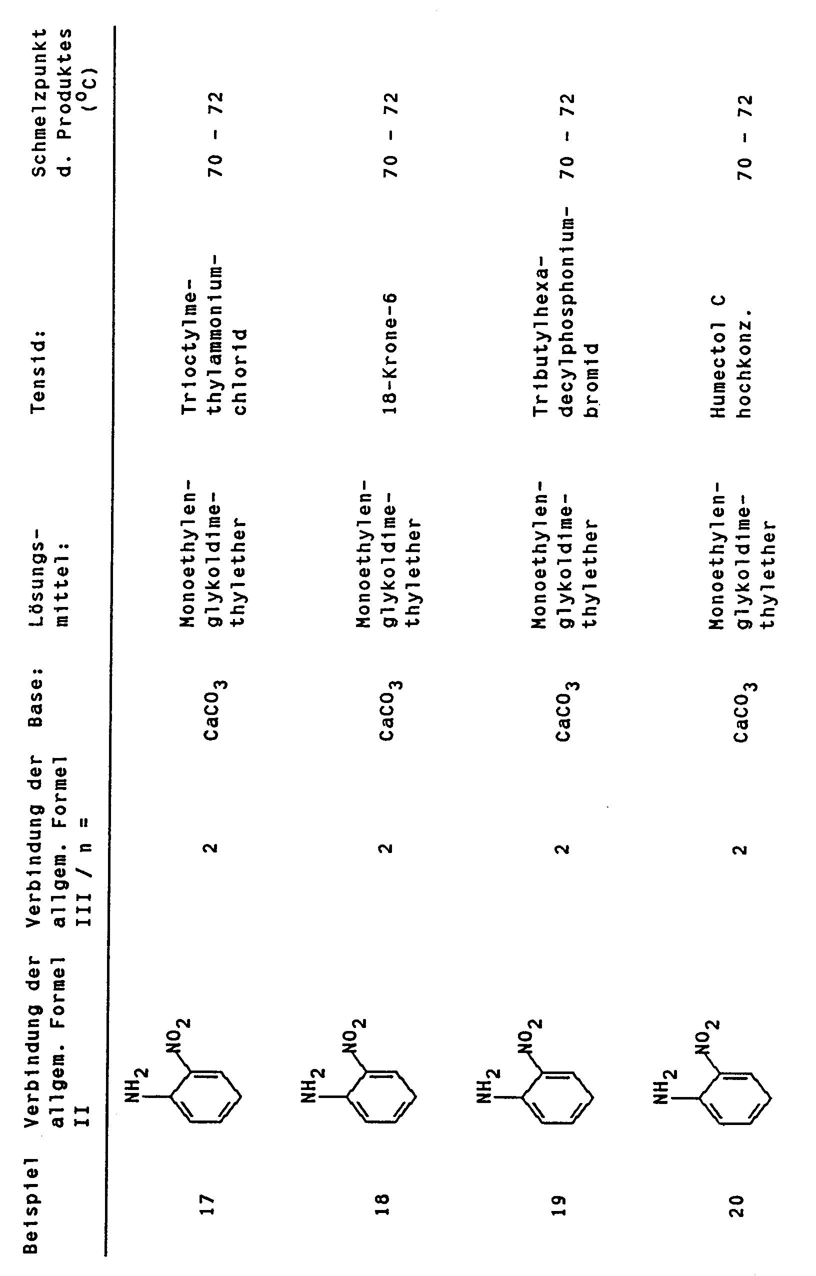

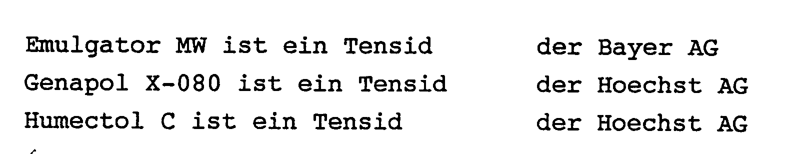

## Patentansprüche

1. Verfahren zur Herstellung von Hydroxyalkylaminonitro-benzolderivaten der allgemeinen Formel I 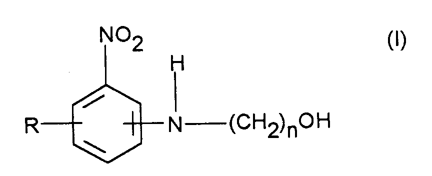 worin
n 2 oder 3; und
R Wasserstoff, Hydroxy, Halogen, (C₁-C₄)-Alkyl, (C₁-C₄)Alkoxy, Amino, Mono-(C₁-C₄)-alkylamino, Di-(C₁-C₄)-alkylamino oder (C₁-C₄)-alkylcarbonylamino
bedeuten,
durch Umsetzung von Verbindungen der allgemeinen Formel II 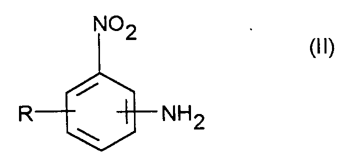 worin R wie oben angegeben definiert ist, mit Verbindungen der allgemeinen Formel III 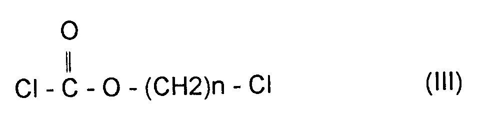 worin n wie oben angegeben definiert ist, in inerten aprotrischen Lösungsmitteln in Gegenwart einer Base zu Verbindungen der allgemeinen Formel IV 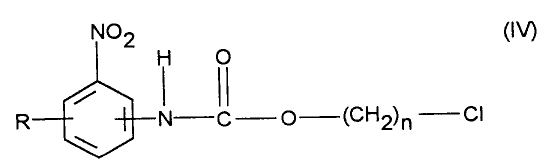 worin R und n wie oben angegebenen definiert sind und Umlagerung der Verbindung der allgemeinen Formel IV mit einem Alkalihydroxid, dadurch gekennzeichnet, daß die Verbindung der allgemeinen Formel IV nicht zwischenisoliert wird und daß nach erfolgter Umsetzung der Verbindung der allgemeinen Formel II mit der Verbindung der allgemeinen Formel III Wasser zugegeben, die salzhaltige Wasserphase bei pH-Werten von 0 bis 7 abgetrennt und die organische Phase weiter zur Verbindung der allgemeinen Formel I umgesetzt wird.

2. Verfahren gemäß Anspruch 1, dadurch gekennzeichnet, daß eine Verbindung der allgemeinen Formel Ia 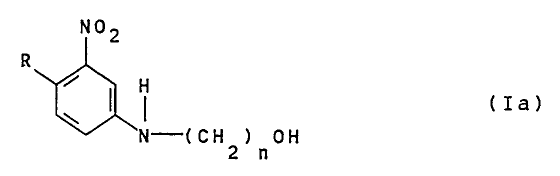 worin R und n wie in Anspruch 1 angegeben definiert sind, hergestellt wird.

3. Verfahren gemäß Anspruch 1 und/oder 2, dadurch gekennzeichnet, daß R Wasserstoff, Methyl, Methoxy, Ethoxy, Hydroxy oder Chlor ist.

4. Verfahren gemäß einem oder mehreren der Ansprüche 1 bis 3, dadurch gekennzeichnet, daß als inerte aprotische Lösungsmittel Diethoxymethan, Diethylenglykoldimethylether, Triethylenglykoldimethylether, Dioxan, Tetrahydrofuran, Monoethylenglykoldiethylether, Monoethylenglykoldimethylether oder Methyl-tert.-butylether eingesetzt werden.

5. Verfahren gemäß einem oder mehreren der Ansprüche 1 bis 4, dadurch gekennzeichnet, daß das inerte aprotische Lösungsmittel 0 bis 30 Gew.% Wasser enthält.

6. Verfahren gemäß einem oder mehreren der Ansprüche 1 bis 4, dadurch gekennzeichnet, daß das inerte aprotische Lösungsmittel 1 bis 10 Gew.% Wasser enthält.

7. Verfahren gemäß einem oder mehreren der Ansprüche 1 bis 6, dadurch gekennzeichnet, daß als Base Alkalicarbonate, Alkalibicarbonate, Erdalkalicarbonate, Erdalkalibicarbonate, Alkalihydroxide, Erdalkalihydroxide, Erdalkalioxide oder tertiäre Amine eingesetzt werden.

8. Verfahren gemäß einem oder mehreren der Ansprüche 1 bis 7, dadurch gekennzeichnet, daß als Alkalihydroxid Lithium-, Natrium- oder Kaliumhydroxid oder deren Mischungen untereinander eingesetzt werden.

9. Verfahren gemäß einem oder mehreren der Ansprüche 1 bis 8, dadurch gekennzeichnet, daß es in Gegenwart von anionischen, kationischen oder nichtionogenen Tensiden oder von Phasentransferkatalysatoren ausgeführt wird.

## Claims

1. A process for the preparation of hydroxyalkylaminonitrobenzene derivatives of the formula I 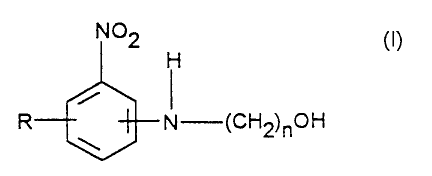 in which
n is 2 or 3; and
R is hydrogen, hydroxyl, halogen, (C₁-C₄)-alkyl, (C₁-C₄)-alkoxy, amino, mono-(C₁-C₄)-alkylamino, di-(C₁-C₄)-alkylamino or (C₁-C₄)-alkylcarbonylamino,
by reacting compounds of the formula II 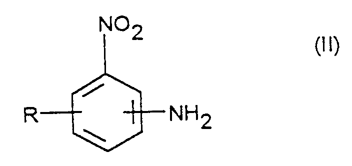 in which R is as defined above, with compounds of the formula III 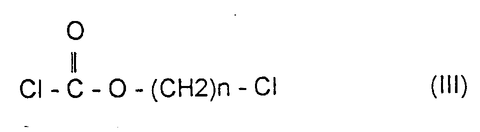 in which n is as defined above, in inert aprotic solvents in the presence of a base to give compounds of the formula IV 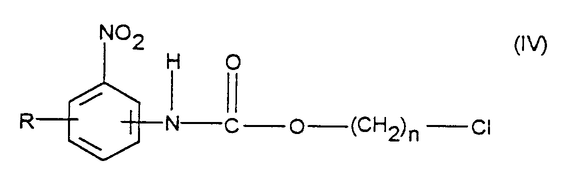 in which R and n are as defined above, and rearranging the compound of formula IV using an alkali metal hydroxide, which comprises not isolating the compound of the formula IV and, following reaction of the compound of the formula II with the compound of the formula III, adding water, removing the salt-containing aqueous phase at a pH of from 0 to 7 and further reacting the organic phase to give the compound of the formula I.

2. The process as claimed in claim 1, which comprises preparing a compound of the formula Ia 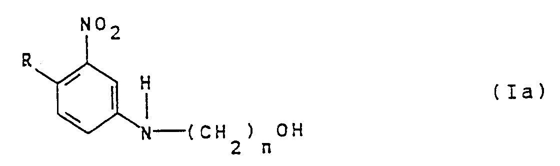 in which R and n are as defined in claim 1.

3. The process as claimed in claim 1 and/or 2, wherein R is hydrogen, methyl, methoxy, ethoxy, hydroxyl or chlorine.

4. The process as claimed in one or more of claims 1 to 3, wherein the inert aprotic solvent used is diethoxymethane, diethylene glycol dimethyl ether, triethylene glycol dimethyl ether, dioxane, tetrahydrofuran, monoethylene glycol diethyl ether, monoethylene glycol dimethyl ether or methyl tert-butyl ether.

5. The process as claimed in one or more of claims 1 to 4, wherein the inert aprotic solvent comprises from 0 to 30% by weight of water.

6. The process as claimed in one or more of claims 1 to 4, wherein the inert aprotic solvent comprises from 1 to 10% by weight of water.

7. The process as claimed in one or more of claims 1 to 6, wherein the base used is an alkali metal carbonate, alkali metal bicarbonate, alkaline earth metal carbonate, alkaline earth metal bicarbonate, alkali metal hydroxide, alkaline earth metal hydroxide, alkaline earth metal oxide or a tertiary amine.

8. The process as claimed in one or more of claims 1 to 7, wherein the alkali metal hydroxide used is lithium hydroxide, sodium hydroxide or potassium hydroxide or mixtures thereof.

9. The process as claimed in one or more of claims 1 to 8, which comprises carrying it out in the presence of anionic, cationic or nonionic surfactants or phase transfer catalysts.

## Revendications

1. Procédé pour la préparation de dérivés d'hydroxyalkylaminonitrobenzène de formule générale I 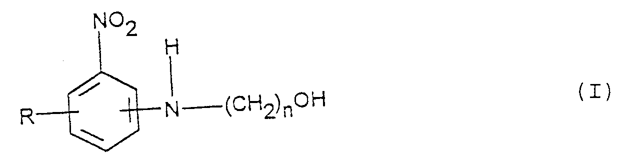 dans laquelle
n vaut 2 ou 3 et
R représente un atome d'hydrogène, un groupe hydroxy, halogène, alkyle en C₁ à C₄, alcoxy en C₁ à C₄, amino, mono-(alkyl en C₁ à C₄)amino, di-(alkyl en C₁ à C₄)-amino ou (alkyl en C₁ à C₄)carbonylamino,
par réaction de composés de formule générale II 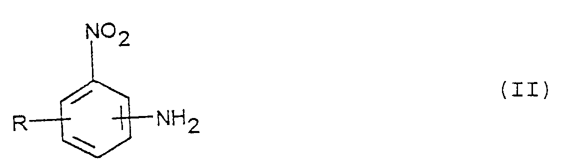 dans laquelle R est défini comme ci-dessus, avec des composés de formule générale III 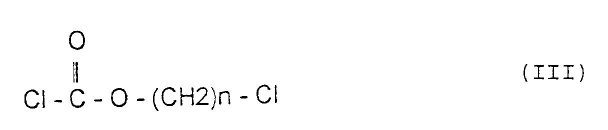 dans laquelle n est défini ci-dessus, en milieu de solvant aprotique, inerte, en présence d'une base pour obtenir des composés de formule générale IV 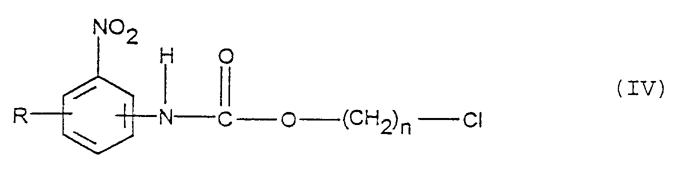 dans laquelle R et n sont définis ci-dessus, et par transposition des composés de formule générale IV avec un hydroxyde alcalin, caractérisé en ce que l'on n'isole pas le composé de formule générale IV de façon intermédiaire, et en ce que, après avoir effectué la transformation du composé de formule générale II avec le composé de formule générale III, on ajoute de l'eau, on sépare la phase aqueuse contenant du sel à des valeurs de pH comprises entre 0 et 7, et on fait réagir la phase organique pour obtenir un composé de formule générale I.

2. Procédé selon la revendication 1, caractérisé en ce que l'on prépare un composé de formule générale Ia 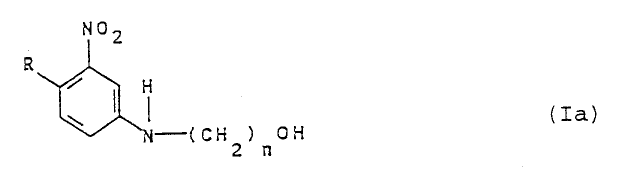 dans laquelle R et n sont définis dans la revendication 1.

3. Procédé selon la revendication 1 et/ou 2, caractérisé en ce que R représente un atome d'hydrogène, des groupes méthyle, méthoxy, éthoxy, hydroxy ou un atome de chlore.

4. Procédé selon une ou plusieurs des revendications 1 à 3, caractérisé en ce que l'on utilise en tant que solvant aprotique, inerte, le diéthoxyméthane, le diméthyléther de diéthylèneglycol, le diméthyléther de triéthylèneglycol, le dioxanne, le tétrahydrofuranne, le diéthyléther de monoéthylèneglycol, le diméthyléther de monoéthylèneglycol ou le méthyl-tert-butyléther.

5. Procédé selon une ou plusieurs des revendications 1 à 4, caractérisé en ce que le solvant aprotique, inerte, contient de 0 à 30 % d'eau.

6. Procédé selon une ou plusieurs des revendications 1 à 4, caractérisé en ce que le solvant aprotique, inerte, contient de 1 à 10 % d'eau.

7. Procédé selon une ou plusieurs des revendications 1 à 6, caractérisé en ce que l'on utilise en tant que bases des carbonates alcalins, des bicarbonates alcalins, des carbonates de métaux alcalino-terreux, des bicarbonates de métaux alcalino-terreux, des hydroxydes alcalins, des hydroxydes alcalino-terreux, des oxydes alcalino-terreux ou des amines tertiaires.

8. Procédé selon une ou plusieurs des revendications 1 à 7, caractérisé en ce que l'on utilise en tant qu'hydroxyde alcalin l'hydroxyde de lithium, de sodium, ou de potassium, ou leurs mélanges.

9. Procédé selon une ou plusieurs des revendications 1 à 8, caractérisé en ce que l'on met en oeuvre le procédé en présence d'agents de surface anioniques, cationiques ou non ionogènes ou de catalyseurs de transfert de phase.
